# EUROPEAN PATENT APPLICATION

(11) **EP 4 548 884 A2**
(43) Date of publication of application: **07.05.2025**
(21) Application number: 25152772.7
(22) Date of filing: 11.12.2020
(51) Int. Cl.: A61F 2/82

(54) **CONNECTING DEVICE FOR MEDICAL DEVICES**

(30) Priority: 13.12.2019 EP 19215990
(62) Divisional of application: 20820963.5
(71) Applicant: Tricares SAS, 75001 Paris (FR)
(72) Inventor: RAHMIG, Georg, 75175 Pforzheim (DE); STRAUBINGER, Helmut, 85609 Aschheim (DE)
(74) Representative: Hoefer & Partner Patentanwälte mbB

(57) **Abstract**

The present invention relates to a novel medical connecting device and medical devices comprising at least one such medical connecting device as well as its use in medical prostheses.

## Description

The present invention relates to a novel medical connecting device and medical devices comprising at least one such medical connecting device as well as its use in medical prostheses.

Minimally invasive techniques and catheter-based implantation techniques have advanced over the years and are now feasible in many medical fields.

In a number of medical fields it is now possible to treat patients by catheter-based techniques allowing for the treatment of such patients who could otherwise not be adequately taken care of due to their physical condition and the risks connected with surgery. Such catheter-based techniques apply to delivery systems, e.g. a catheter and/or introducer sheath, for implanting the medical device to a desired target site via different access routes into a patient's body.

In such procedures the prostheses used are made of one part or are composed of two or multiple parts. The parts can be sewed together or be attached to each other by fitting two parts into each other. It is also possible to assemble the different parts in vivo during the deployment procedure. The correct fitting and durability is an important aspect in such medical devices.

In particular, in recent years the treatment of heart valve diseases and deficiencies has become more and more successful. Examples are trans-apical, trans-jugular and trans-femoral procedures for heart valve replacement therapies, e.g. aortic or mitral heart valve treatments.

In many cases a stent-based prosthesis with a tissue based replacement valve is used and implanted to replace the native heart valve using a catheter delivery system.

The replacement heart valve prosthesis has to be crimped and loaded onto the catheter. Such a prosthesis can be composed of one single part or it can be composed of two or multiple parts. One aspect in such a prosthesis is the connection of the various parts to form one functional prosthesis before or upon implantation in a patient.

More particularly, it is a problem to provide for a easy to use, reliable connecting means useful for medical devices and/or exhibiting dimensions which comply well with catheter based delivery methods, or to provide for connecting means useful for medical devices with improved features vis-à-vis the state of the art.

Hence there exists a need for an easy to use and reliable connecting means useful in connecting two medical device parts or a number of medical device parts which preferably comply with the needs of catheter based delivery methods.

Accordingly, it is one object of the current disclosure to provide a reliable connecting means to combine medical device parts in an easy and/or efficient and/or reliable manner and/or compatible with catheter crimping and/or catheter delivery, or at least to provide a connecting means wherein the disadvantages of the prior art are essentially avoided or which show reduced disadvantages vis-à-vis the prior art.

### Brief Summary of the Disclosure

In one aspect the disclosure relates to a medical connecting device useful for connecting two or multiple medical device parts characterized by engaging and locking two connecting features of two medical device parts wherein a first connecting feature forms part of a first medical device and a second connecting feature forms part of a second medical device wherein the medical connecting device is comprising or exhibiting at least one snap lock tongue and one snap lock cover.

In another aspect the disclosure relates to a two part medical device comprising a first part and a second part wherein each part comprises at least one connecting features wherein one connecting features of the first part and one connecting features of the second part are aligned in the same direction or in a counter direction and a snap lock cover is engaging the two connecting features and the two connecting features are locked by way of at least one snap lock tongue, preferably one or two snap lock tongues, and at least one strut tongue stop of each connecting feature wherein also multiple medical device parts can be connected.

In another aspect the disclosure relates to a two part stent comprising an inner stent and an outer stent wherein each stent comprises at least one connecting features wherein one connecting feature of the first stent and one connecting feature of the second stent are aligned in the same direction or in a counter direction and a snap lock cover is engaging the two connecting features and locking the two connecting features by way of at least one snap lock tongue, preferably one or two snap lock tongues, and at least one strut tongue stop of each connecting feature wherein also multiple medical device parts can be connected.

In another aspect the disclosure relates to a heart valve replacement prosthesis comprising a replacement valve and a two part stent comprising an inner stent and an outer stent wherein each stent comprises at least one connecting feature wherein one connecting feature of the first stent and one connecting feature of the second stent are aligned in the same direction or in a counter direction and a snap lock cover is engaging the two connecting features and locking the two connecting features by way of at least one snap lock tongue and one strut tongue stop of each connecting feature.

In another aspect the disclosure relates to method for assembling two or more medical device parts, e.g. stent parts, using one or more medical connecting devices according to the disclosure to obtain an assembled medical device prosthesis.

In another aspect the disclosure relates to a the use of one or more medical connecting devices according to the disclosure to connect different medical device parts.

### Brief Description of the Drawings

Various embodiments of the disclosure are exemplified by the Figures wherein:
Fig. 1 illustrates one embodiment of a connecting device according to the disclosure.
Fig. 2, 2a, 2b illustrate details of various possibilities of connecting two medical parts with each other according to the disclosure.

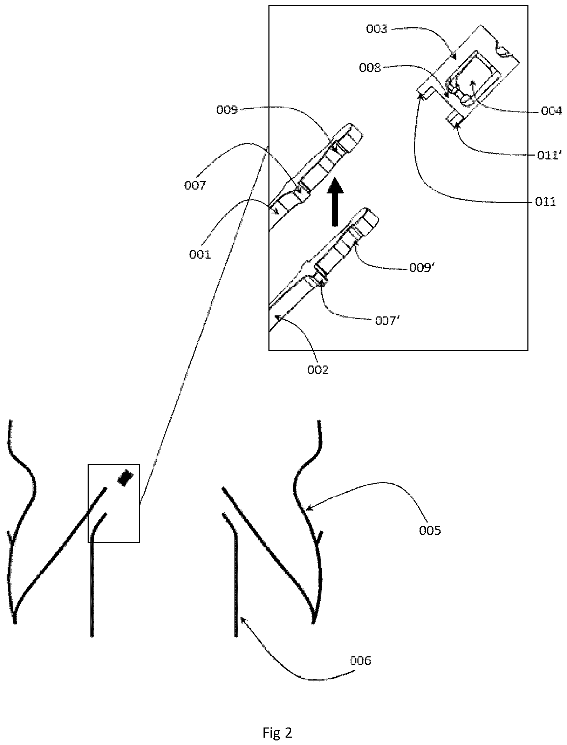

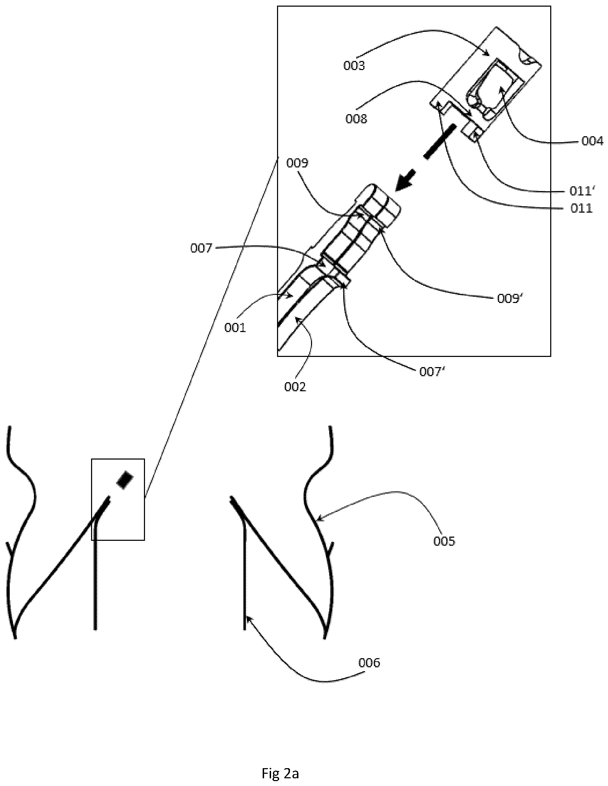

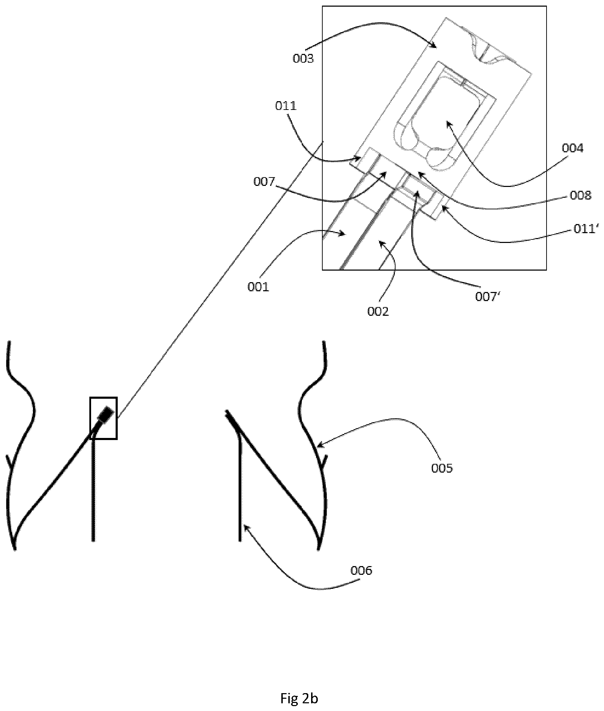

Fig. 2 to 4 show various snap lock covers according to the disclosure.

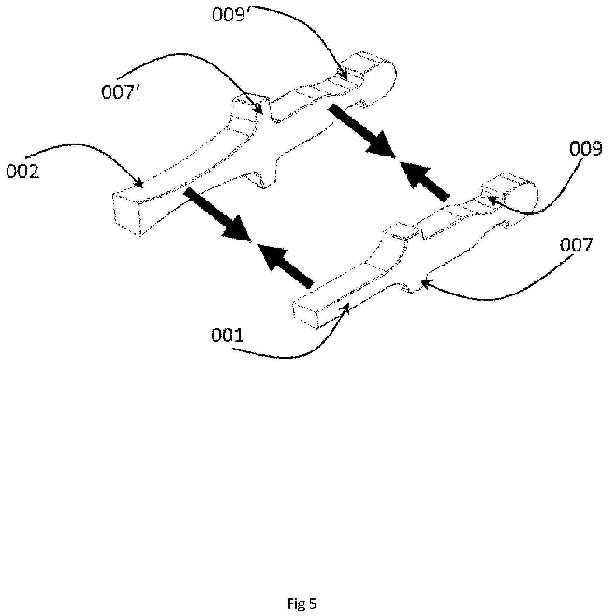

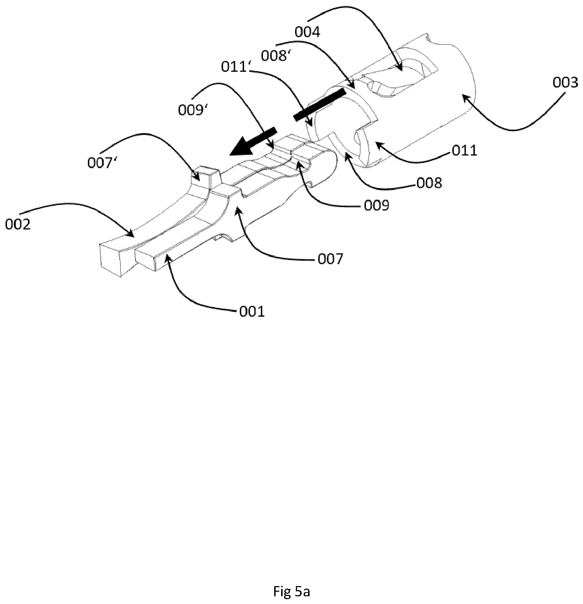

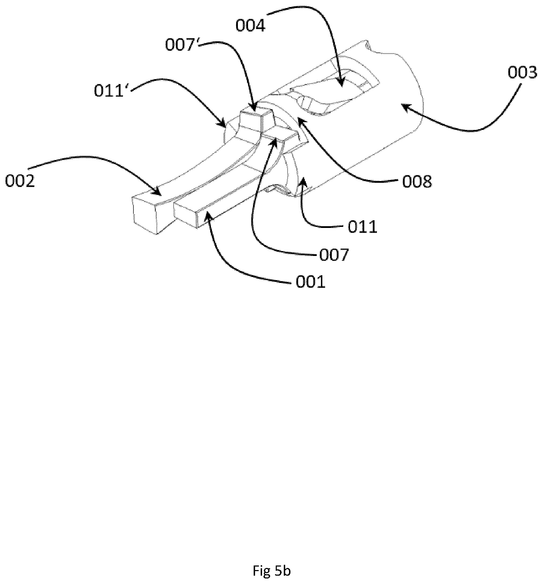

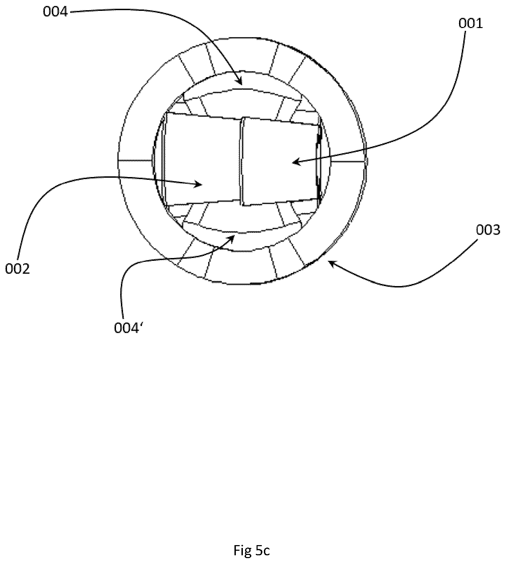

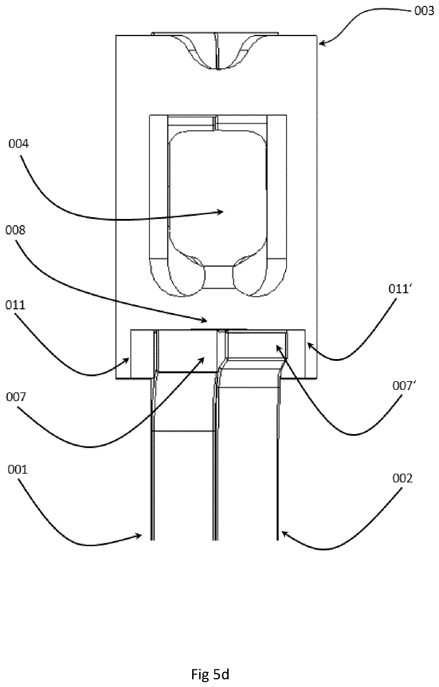

Fig. 5, 5a to 5d depict various possibilities of strut alignment and snap lock cover connection according to the disclosure.

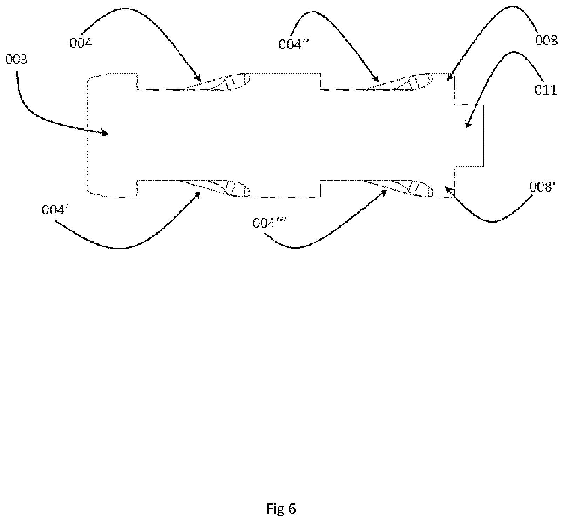

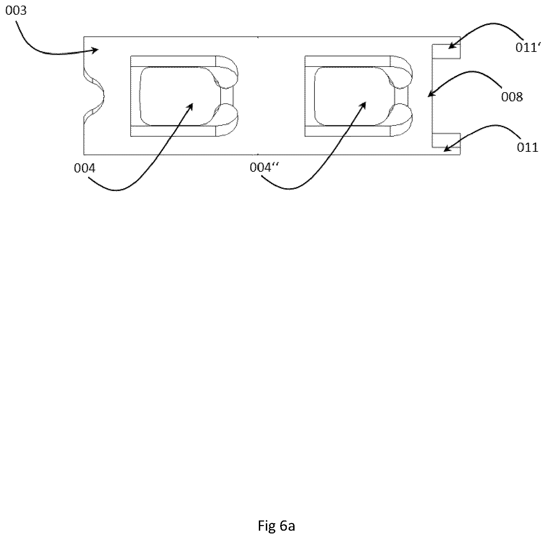

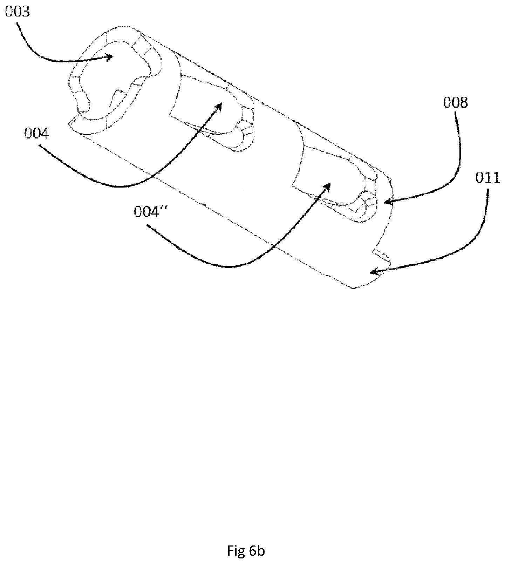

Fig. 6 to 8 show various embodiments of snap lock covers according to the disclosure.

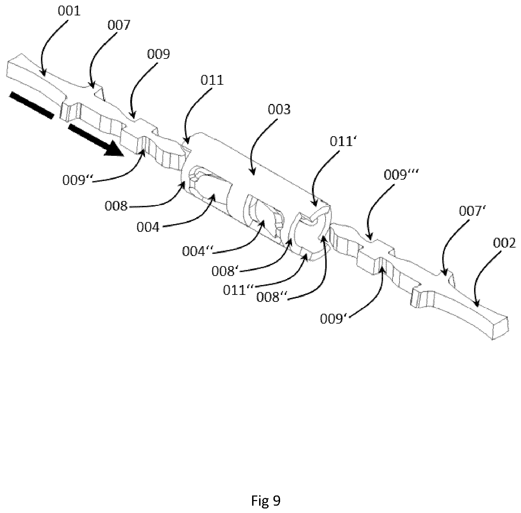

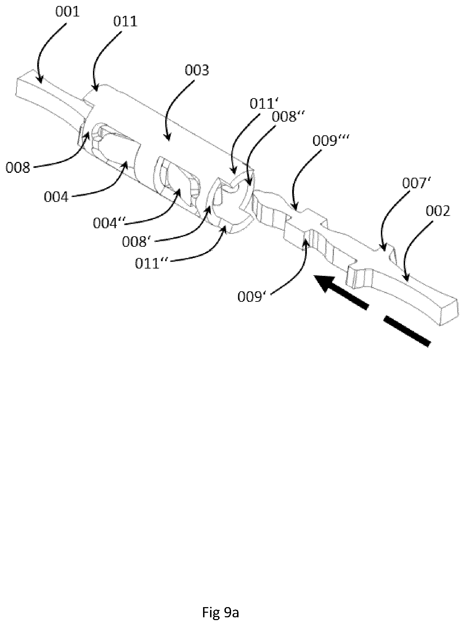

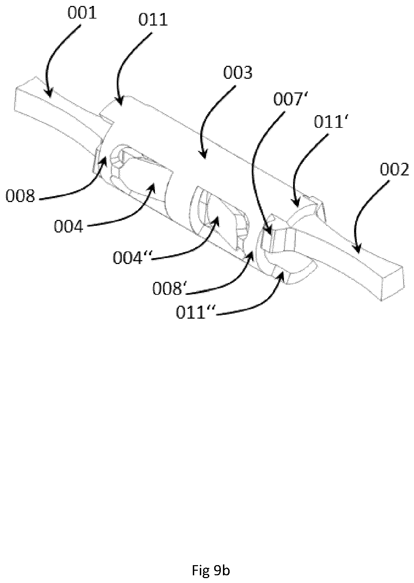

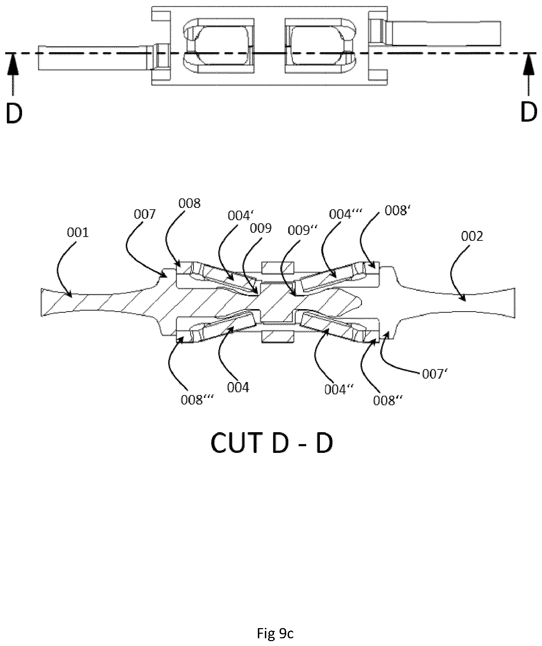

Fig. 9, 9a to 9c show different embodiments according to the disclosure of connecting struts of different medical device parts using a snap lock cover according to the disclosure.

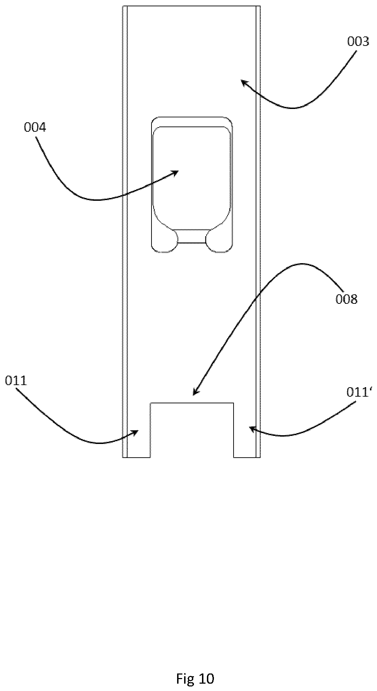

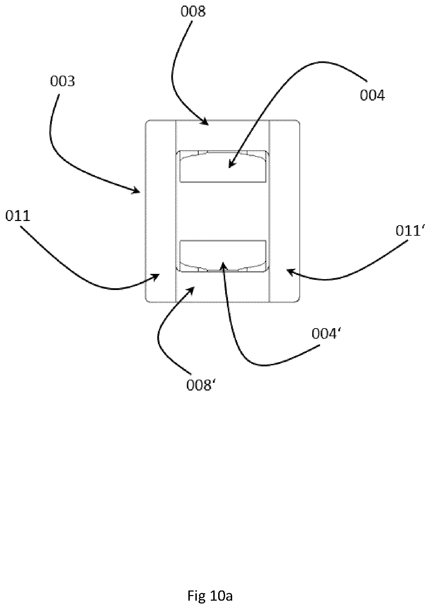

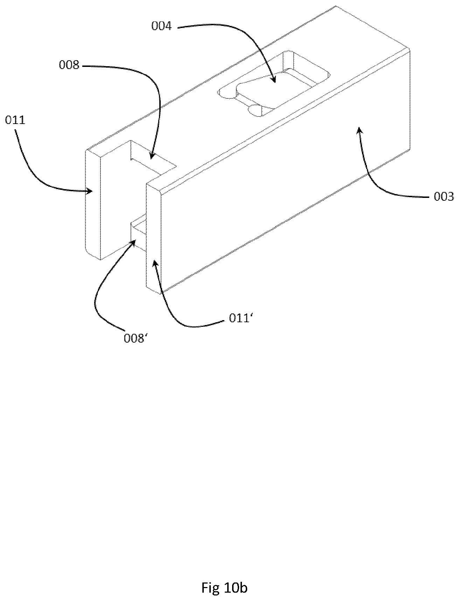

Fig. 10, 10a, 10b illustrates different embodiments of snap lock covers according to the disclosure.

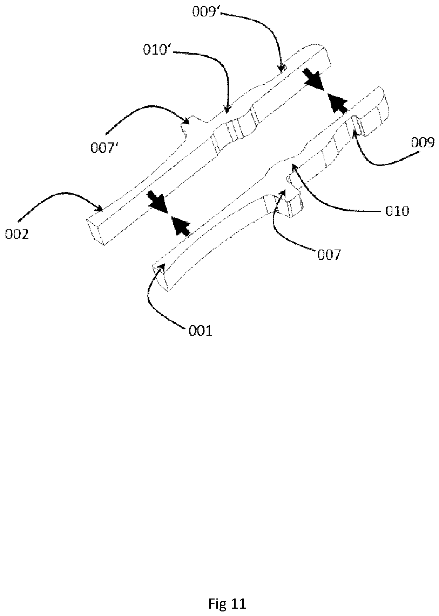

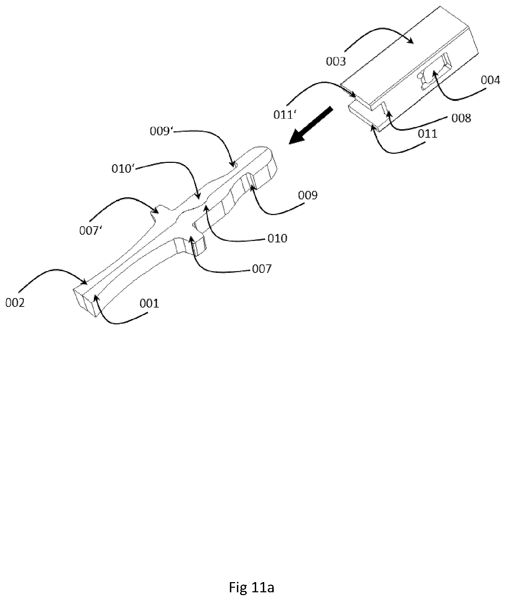

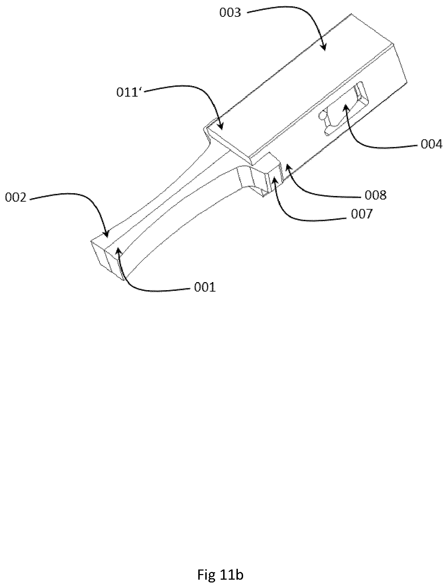

Fig. 11 to 16b show different embodiments of snap lock covers according to the disclosure including stop variations and different possibilities of strut alignments according to the disclosure.

### Detailed Description

In the following certain terms of the disclosure will be defined. Otherwise technical terms in the context of the disclosure shall be understood as by the applicable skilled person.

The term "prosthesis" or "medical device" or "implant" in the sense of the disclosure is to be understood as any medical device that can be delivered in a minimally invasive fashion or by way of a catheter based procedure. The terms can be used interchangeably. A prosthesis in the sense of the disclosure can be e.g. a stent or stent-based prosthesis or stent-based replacement heart valve prosthesis like an aortic replacement heart valve, a mitral replacement heart valve or a tricuspid replacement heart valve.

The term "catheter" or "delivery device" in the sense of the disclosure is to be understood as the device used to deploy a prosthesis in a patient at a determined site, e.g. to replace a heart valve like a native aortic heart valve, mitral heart valve or tricuspid heart valve.

A "mesh stent" or "braided mesh stent" or "braided stent" in the sense of the disclosure is a stent composed of wires in contrast to e.g. a laser cut nitinol tube.

A "cut stent" or "laser cut stent" in the sense of the disclosure is a stent which is laser cut from e.g. a nitinol tube. Due to its tube origin the outside radius is larger than the inside radius and thus when cut into a stent is tapered from the outside to the inside (original tube).

A "stent area" or "stent areas" in the sense of the disclosure is a defined area of the outer stent, mesh stent or the replacement heart valve prosthesis and in particular it is a longitudinal section or an outer section defined as proximal, middle or distal area or atrial, annular or ventricular.

A "proximal area", "middle area", "distal area" in the sense of the disclosure denotes areas of the stent or prosthesis in relation to the operator performing implantation by use of a catheter wherein proximal is close to the operator and distal is away from the operator. "Middle area" denotes in a stent or prosthesis in the sense of the disclosure is the area between the distal and proximal area. The "proximal area" can also be denoted inflow end or inflow area and the "distal area" can also be denoted outflow end or outflow area with regards to the natural blood flow in situ, i.e. in vivo, in an individual (person or patient); proximal can also be denoted atrial, middle can be denoted annular and distal can be denoted ventricular.

An "annulus area" or "annular area" in the sense of the disclosure is either the respective area of an endogenous heart valve or it defines the respective area in the replacement heart valve or stent which is to be positioned at the implantation site and it meant to align with the endogenous annulus.

A "sub-annular area" in the sense of the disclosure is the area of the prosthesis which is in distal direction (or in inflow direction or in ventricular direction) of the annulus of the endogenous heart valve. The prosthesis may cover the "sub-annular area" with the U or V grove area and the distal area.

A "groove" in the sense of the disclosure describes an area of the stent or of the prosthesis exhibiting a smaller diameter than other areas and wherein distally and proximally of said groove other areas of the stent or prosthesis having a larger diameter are in neighborhood to said groove; said groove can have a V or U shape or combinations thereof or any other bended and useful geometries or it can be characterized by just a smaller diameter as compared to the atrial and ventricular stent areas.

A "multi-part stent" in the sense of the disclosure can refer to a "two-part stent" or a "three-part stent" wherein the inner stent is connected by way of a medical connection device according to the disclosure. The medical connection device according to the disclosure can be positioned at the atrial end or area of the inner stent or in the annulus areas of the native valve.

A "target site" in the sense of the disclosure is the location or place where the replacement heart valve prosthesis is to be implanted and where a dysfunction or malfunction shall be treated, e.g. at the annulus of a tricuspid or mitral heart valve.

A "connection" of the stents in the sense of the disclosure is the way of fixation of one medical connection device according to the disclosure of two medical device parts wherein several medical device parts can be assembled in this way to a final multiple part medical device ready for implantation or assembled during implantation in a patient.

"Fixation means" or "anchoring means" in the sense of the disclosure is a component connected with the inner stent and providing essentially for anchoring the prosthesis at the target site, optionally in cooperation with additional means. In a particular aspect a fixation means is composed of or comprises an anchoring loop, an anchoring arm, a connecting arch and an inner stent anchor, preferably a connecting means connects the inner stent anchor of the fixation means with the inner stent.

An "anchoring loop" in the sense of the disclosure is a part of a stent useful in fixation of a stent or prosthesis and which aids in avoiding movement of the stent or prosthesis at the target site. In general an anchoring loop in the sense of the disclosure is a means useful for an improved fixation of a stent or prosthesis wherein a loop is fixed to or connected with or forms part of or is an integral part of the inner stent. The "loop" or "loops" in the sense of the disclosure can have different shapes like round, square etc. and are located in a defined area in a defined pattern. A "loop" in the sense of the disclosure will exhibit a defined angle with regard to the inner stent surface and it may be designed that it may stretch out straight or flip over when the stent or prosthesis is retrieved into the catheter after an initial and possibly partial deployment.

An "angle structure" or "angle" in the sense of the disclosure is an angle between two accessory lines drawn at a certain area or stent layer in order to define a certain geometry of said stent part or arch or layer with regard to other stent structures like the inner stent.

A "radial force" in the sense of the disclosure is the force exhibited by a stent or prosthesis in radial outward direction, more particularly the force exhibited by the outer stent of the prosthesis which may be a mesh or a laser cut stent, e.g. a nitinol stent. The radial force depends on the particular mesh or cut stent design and relates to the material density, e.g. the density of wires per square area in a mesh stent, or the number of cells and size of said cells circumferentially in a certain laser cut stent level or area, e.g. the proximal/atrial, middle/annular or distal/ventricular area. The radial force in a replacement heart valve prosthesis according to the disclosure will be chosen for the outer stent or the combination of the inner and outer stent and fixation means in a magnitude to provide for good contact with the surrounding tissue and to support the fixation functionality of the stent or prosthesis and optionally also as little as possible in order to avoid an interference with the endogenous environment and biology of the target site. Thus it will be chosen in a magnitude in order to avoid interference with the implantation site and endogenous tissue and function. The radial force may be supported for its fixation function by other means, e.g. loops for fixation.

The "target area" in the sense of the disclosure is the three-dimensional space surrounding or being within the native organ like a native heart valve which can be e.g. a tricuspid or mitral heart valve.

An "atraumatic design" of the loops in the sense of the disclosure is wherein the loops or other means or parts of a stent or a prosthesis are designed to avoid any or essentially any damage of the surrounding tissue or tissue in contact with said parts or at least parts manufactured in a manner to minimize damaging or/and injuring the tissue which they contact.

"Compliance" of the stent or replacement heart valve prosthesis, e.g. comprising an inner laser cut stent within an outer mesh stent, or a laser cut inner stent within a laser cut outer stent, in the sense of the disclosure relates to a positive interference with the target tissue. "Compliance" relates to a design which exhibits good geometry adaptation of the stent or prosthesis to the implantation site and wherein the stent or prosthesis exhibits advantageous fixation characteristics, good functionality as concerns valve function and at the same a minimal interference with the endogenous heart structures and heart function.

"Medical device" in the sense of the disclosure can mean any part or assembly of parts which can be used in the medical context or a medical application. It can relate to surgery devices or micro invasive tools or medical devices designed for the implantation of delivery into a patient by way of surgery of by way of minimally invasive techniques of using a delivery system like a catheter. In particular "medical device" can relate to stents, replacement heart valve prostheses, repair devices or replacement devices in general, e.g. aortic, pulmonary, mitral, tricuspid replacement heart valve prostheses.

A "medical connecting device" in the sense of the disclosure means a device which can connect two parts of a medical device minimally using two connecting means (e.g. struts) and a cover, e.g. a snap lock cover, which comprises a means to releaseably or non-releaseably connect the two connecting means. A medical connection device according to the disclosure can be used to connect multiple medical device parts to form a final medical device ready for use in a patient or all or parts thereof can be connected during the delivery procedure, e.g. by way of catheter. Thus one, two, three, four, five, six or more medical connection device according to the disclosure can be useful to provide a multiple part medical device which parts are produced separately and connected by this way lateron.

A "snap lock cover" in the sense of the disclosure means a part which can releaseably or non-releaseably connect two connecting means like struts of a stent or a replacement heart valve prosthesis. It can comprise one or more snap lock tongues, one or more snap lock hard stops, one or more snap lock cover skirt.

A "snap lock tongue" in the sense of the disclosure means a part forming part of the snap lock cover useful to engaging a connecting means like a strut and holding e.g. two struts in a predefined position and thus connecting two medical parts. The snap lock tongue has a counterpart in each strut and thus can hold and fix two struts together.

A "snap lock hard stop" in the sense of the disclosure means a predefined stop within the snap lock cover for engaging the strut.

A "strut tongue stop" in the sense of the disclosure means is the counterpart comprised in the strut or any connecting means that can engage with the snap lock tongue to form a connection and to connect together two medical parts and their connecting means respectively.

A "connecting feature" in the sense of the disclosure means is a part of a medical device part which is meant to engage in the snap lock cover.

A "snap lock cover skirt" in the sense of the disclosure means is a part of the snap lock cover which is useful to support the alignment of, e.g. two struts, and add in the connection and correct positioning of the connecting means in the snap lock cover.

"Distal" in the sense of the disclosure means away from the operator when using a catheter means.

"Proximal" in the sense of the disclosure means close to the operator when using a catheter means.

"Longitudinal" or "longitudinal direction" in the sense of the disclosure means in the longitudinal direction of a medial device, e.g. a heart valve replacement prosthesis.

"Inwardly in an angle" in the sense of the disclosure means e.g. in a valve or replacement heart valve in direction to the middle part or inside part or region of the device in contrast to the outside part or region.

"Round in shape", "square-cut in shape" or "angular in shape" in the sense of the disclosure relates to the design of the snap lock cover. A square cut shape or angular shape can be advantageous because it may use less space and thus be preferred in a small context of a medical device implanted by way of a catheter delivery and the need to crimp the device like a replacement heart valve prosthesis.

The term "position" or "positioning" in the sense of the disclosure relates in general to the broader orientation of the two medical device parts which shall be connected according to the disclosure. The term can also make reference to the orientation in space with regards to the operator who is operating the delivery or deployment device in the sense of proximal (close to the operator / handle of the catheter) and distal (away from the operator / handle of the catheter). In the context of a heart valve replacement prosthesis it refers to the longitudinal shape of the prosthesis which is implanted at a native heart valve site by way of a catheter procedure.

To "align" or "alignment" of parts in the sense of the disclosure means the micro positioning of two connecting means next to each other and the micro positioning of the two connecting means in a manner to fit into the snap lock cover. Hence the orientation of the struts of the two medical device parts at a very close distance in the context of the snap lock cover and its connection therewith. Preferably the alignment of two struts (connecting means) can be supported by strut align means in order to have the two struts in a very exact alignment before or during connection with the snap lock cover.

The alignment of struts and snap lock cover can be described in relation to the original tube wherefrom the medical device parts have been cut (laser cutting method) or in relation to the orientation in which they are positioned in space during the catheter delivery and deployment procedure in relation to the operator, i.e. proximal to the operator (with respect to the handle of the catheter) or distal to the operator (with respect to the handle of the catheter).

To align "up-to-down" or "down-to-up" (the two terms relate to the point of view of the viewer) or "superimposed" in the sense of the disclosure means the positioning of two connecting means to each as described in Fig. 2. The struts (connecting means) are superimposed in relation to the proximal-distal orientation of the prosthesis as depicted in Fig. 2.

To align "tube cut edge to tube cut edge" of the connecting feature(s) of the inner part or stent in the sense of the disclosure makes also reference to the method of production and laser cutting of the medical parts in question wherein the tube (e.g. nitinol tube) has an inside and an outside surface of the tube and the edges where the tube was cut. Making reference to the original tube from which the medical part, e.g. stent, is cut, one can define the alignment according to the disclosure inside and outside of the tube and thus of the medical part (e.g. stent) and on the other hand the cut level and cut side or area. Thus one can define outside-to-inside alignment of two parts, wherein depending on the view point this can mean inside-to-outside or outside-to-inside (which means the same just is different because of a different view point), or outside-to-outside, or inside-to-inside; side-by-side can mean cut side-to-cut side.

Another way to define the orientation of the medical device parts can be in relation to the operator using a catheter, i.e. proximal and distal in relation to the operator and the handle of the catheter, respectively.

To align the connecting feature(s) of the inner part or stent "side-by-side" in the sense of the disclosure means that two struts of a two part stent (or two part heart valve replacement prosthesis) are positioned next to each other in relation of the longitudinal direction of the device which can also be expressed as the proximal to distal direction taking as reference the view point of the operator in case of a catheter based delivery procedure. Thus the struts of a prosthesis are at the same level in relationship to the proximal to distal orientation and in relation to the operator.

In "directly inward" direction in the sense of the disclosure means that a part, like a snap lock tongue, is not pointing with its outmost edge 100 % in the longitudinal direction but with a certain angle in the inward direction with relation to the longitudinal direction of the device (003). The angle can vary as useful and thus may have a degree of between 5° to 75°.

To "engage" or "engaging" in the sense of the disclosure means that two means for connecting and/or locking are brought into close contact in order to connect the two medical device parts.

To "lock" or "locking" in the sense of the disclosure means that two medical device parts are connected by features designed to engage with a snap lock cover.

"Snap lock cover" in the sense of the disclosure means a part which can functionally connect two means of two medical device parts and facilitate their connection in a stable and secure manner.

"Snap lock cover skirt" in the sense of the disclosure means a feature which supports a secure locking in the sense that it adds stability on two sides by way of extending the snap lock cover. Moreover the snap lock cover skirt in combination with the strut hard stop advantageously reduces or essentially entirely avoids tilting of the struts within the snap lock cover and thus unwanted decoupling of struts and snap lock cover.

"Strut align means" in the sense of the disclosure means serves to facilitate the alignment of two connecting means (e.g. struts) of two medical device parts as illustrated in Fig. 11. The strut align means do not only advantageously provide for correctly assembling of the struts for correct positioning and locking into the snap lock cover but avoid sliding of the struts against each other and thus avoid fretting or/and material fatigue. This can be very advantageous in an embodiment of side-by-side strut alignment and I the context of a heart valve replacement prosthesis wherein very high forces occur in the patient and during heart beating.

"Two strut align means of connecting feature pairs" or "connecting feature pair" in the sense of the disclosure means that the pair is part and counterpart used for connecting two medical device parts by way of a Medical connecting device according to the disclosure. This can facilitate the alignment and durability of the device.

"Two part" or "two part medical device" in the sense of the disclosure means that in each case two parts are connected with one medical connecting device according to the disclosure wherein multiple parts (2, 3, 4, 5, 6 or more) can be connected to form a functional multiple part medical device.

In the following various aspects of the disclosure are described.

In one aspect of the disclosure, a problem underlying the application is solved by a medical connecting device useful for connecting two or multiple medical device parts characterized by engaging and locking two connecting features of two medical device parts wherein a first connecting feature forms part of a first medical device and a second connecting feature forms part of a second medical device wherein the medical connecting device is comprising or exhibiting at least one snap lock tongue and one snap lock cover.

The medical connecting device advantageously provides for a fast, easy and reliable means to connect two or more parts of a medical device, In addition it may be possible to connect said medical device parts before or during implantation or during the delivery process or deployment procedure.

The different medical device parts need solely to be connected and automatically form a correct and firm connection of the different parts. Due to its construction it is also a reliable and a safe connection which does not imply tissue damages because of the non-invasive and advantageous shape of the snap lock cover which avoids injuries of surrounding tissue.

Moreover, an additional advantage is that the connection does not imply additional assembling steps or material like in welding, suturing, or gluing.

In addition, with the advantageous medical connecting device according to the disclosure the materials of the medical parts and its materials are not subject to mechanical, thermal, chemical or other stress which may have a negative impact on the material and durability of the device when in use in a patient.

Finally, the advantageous medical connecting device according to the disclosure implies faster production and assembly, reduced material and process time and thus less cost. Hence, the medical connecting device according to the disclosure is economically advantageous.

The use of a medical connecting device according to the disclosure and also be reopened with special tolls which is not possible with know mechanical of chemical connection approaches.

A medical connecting device according to the disclosure can exhibit variations in its design and can be adapted to the particular application used for, e.g. a stent, replacement heart valve prosthesis.

In certain aspects it can be preferred if the medical connecting device according to the disclosure is modified in that the snap lock cover comprises further at least one snap lock hard stop, preferably 2, 3, 4, 5, 6, 7, or 8, and/or at least one snap lock cover skirt, preferably 2, 3, or 4. Thus advantageously the position of connecting struts can be more precisely adjusted and the connection of the two or more different medical device parts is very precisely aligned as desired.

A snap lock hard stop or more snap lock hard stops in a medical connecting device according to the disclosure can be positioned as is useful and in agreement with the other design features thereof. A medical connecting device according to the disclosure can be designed so that one or several snap lock hard stops are positioned at the proximal end, or at the distal end or/and in a region between the distal and proximal end of the snap lock cover.

The snap lock cover of a medical connecting device according to the disclosure is not limited in its geometrical dimensions or design and can be adapted according to the needs of the other features and under functional and technical considerations. A snap lock cover according to the disclosure may be e.g. a medical connecting device wherein the snap lock cover is round or square-cut or angular in shape or/and longitudinal in shape.

In another aspect of the disclosure, a problem underlying the application is solved by a two part medical device comprising a first part and a second part wherein each part comprises at least one connecting features wherein one connecting features of the first part and one connecting features of the second part are aligned in the same direction or in a counter direction and a snap lock cover is engaging the two connecting features and the two connecting features are locked by way of at least one snap lock tongue, preferably one or two snap lock tongues, and at least one strut tongue stop of each connecting feature wherein also multiple medical device parts can be connected.

In another aspect of the disclosure, a problem underlying the application is solved by a two part stent comprising an inner stent and an outer stent wherein each stent comprises at least one connecting features wherein one connecting feature of the first stent and one connecting feature of the second stent are aligned in the same direction or in a counter direction and a snap lock cover is engaging the two connecting features and locking the two connecting features by way of at least one snap lock tongue, preferably one or two snap lock tongues, and at least one strut tongue stop of each connecting feature wherein also multiple medical device parts can be connected.

In another aspect of the disclosure, a problem underlying the application is solved by a heart valve replacement prosthesis comprising a replacement valve and a two part stent comprising an inner stent and an outer stent wherein each stent comprises at least one connecting feature wherein one connecting feature of the first stent and one connecting feature of the second stent are aligned in the same direction or in a counter direction and a snap lock cover is engaging the two connecting features and locking the two connecting features by way of at least one snap lock tongue and one strut tongue stop of each connecting feature. The number of snap lock tongues and respective strut tongue stops, strut hard stops, snap lock hard stops can be adapted as useful for the respective application and can for each means be 1, 2, 3, 4, 5, or 6.

It can be advantageous if the struts are aligned in a way so that the tube cut edges - due to the tube origin - are tapered from the outside to the inside direction with regards to the original tube are aligned so that the tapering is oriented in counter direction for an improved alignment of the areas next to each other. This may imply less fretting and abrasion during heart valve movement in the heart context and when the heart is in action.

A heart valve replacement prosthesis according to the disclosure and as described above implies the advantage that its materials are not subject to mechanical, thermal, chemical or other stress which may have a negative impact on the material and durability of the device when in use in a patient. In particular in the context of patient implantation in the heart high forces occur and a superior durability is necessary.

In general the below variations of features apply equally to a two part medical device or two part stent or heart valve replacement prosthesis which may be composed of two or multiple parts.

In one embodiment of a two part medical device or two part stent or heart valve replacement prosthesis according to the disclosure it may be advantageous wherein the connecting feature(s) of the inner part or stent is (are) directing inwardly in an angle with regard to the longitudinal direction of the inner part or stent of about 3° to 60°, preferably of 5° - 30°, more preferably 25°.

The parts to be connected can have special struts or otherwise means for connecting the two parts by way of a snap lock cover and which are suitable to be connected by way of a snap lock cover and fixed thereby. Thus the two parts are correctly aligned and reliably connected to form the final medical device as desired. E.g. two parts can be positioned within each other to form the final medical device. In a preferred embodiment the two part medical device or two part stent or heart valve replacement prosthesis according to the disclosure can advantageously be designed wherein the connecting feature(s) of the outer part or stent is (are) superimposed (up-to-down aligned) in relation to the in distal to proximal orientation or aligned inside-to-outside, or inside-to-inside, or outside-to-outside side-by-side with reference to the original tube from which the part or stent was cut, or aligned tube cut edge to tube cut edge with the connecting feature(s) of the inner part or stent.

Thus depending on the design of the single parts of the medical device or/and the relative positioning of these parts to each other the connecting means, e.g. two struts (each strut or connecting means from one medical device part), can be positioned and aligned to each other as it will best fit the final design of the assembled medical device.

According to the disclosure the particular parts and means of the medical connecting device or/and in the two part medical device or two part stent or heart valve replacement prosthesis can be varied depending to the technical advantages or/and needs. Thus the single features snap lock cover, snap lock tongue, snap lock hard stop, strut hard stop, strut tongue stop, strut align means, snap lock cover skirt and/or struts (connecting struts or means) can have different designs, numbers as is required under the particular application. A medical connecting device or/and in the two part medical device or two part stent or heart valve replacement prosthesis according to the disclosure may preferably comprise one snap lock cover and 2, 3 or 4 snap lock tongues and the respective numbers of strut tongue stops of the connecting features.

In another preferred embodiment according to the disclosure a two part medical device or two part stent or heart valve replacement prosthesis may comprise 1 snap lock hard stop and one strut hard stop of each connecting feature or, 2, 3 or 4 snap lock hard stops and 1 or 2 strut hard stops of each connecting feature.

In another preferred embodiment according to the disclosure a two part medical device or two part stent or heart valve replacement prosthesis is characterized in that the snap lock tongue(s) is/are positioned in longitudinal direction of the medical device, stent or prosthesis or essentially 90° to the longitudinal direction or in an angle of between 5° and 90°, or 10° to 45° or 45° to the longitudinal direction.

In another preferred embodiment according to the disclosure a two part medical device or two part stent or heart valve replacement prosthesis is characterized in that the connecting feature is positioned in the distal area or at the distal end of a strut extending from each part of the medical device, stent or prosthesis.

In another preferred embodiment according to the disclosure a two part medical device or two part stent or heart valve replacement prosthesis is characterized in that the connecting feature or each connecting feature comprises at least one strut align means, preferably wherein two strut align means of connecting feature pairs.

In another preferred embodiment according to the disclosure a two part medical device or two part stent or heart valve replacement prosthesis is characterized in that it comprises at least one medical connecting device according to the disclosure.

In another aspect of the disclosure, a problem underlying the application is solved by a method for assembling two or more medical device parts, e.g. stent parts, using one or more medical connecting devices according to the disclosure to obtain an assembled medical device prosthesis.

In particular for assembling the medical connecting device according to the disclosure and a respective method for assembling the medical parts has the advantage that the connection does not imply additional assembling steps which take time and/or materials like in welding, suturing, or gluing.

In another aspect of the disclosure, a problem underlying the application is solved by the use of one or more medical connecting devices according to the disclosure to connect different medical device parts.
he use of a medical connecting device according to the disclosure provides for an easy, reliable and cost effective approach vis-à-vis the known approaches of the prior art and is thus superior compared thereto.

### Examples

The following examples serve to illustrate various embodiments of the disclosure. The examples are not meant to be interpreted as restrictive in any way.

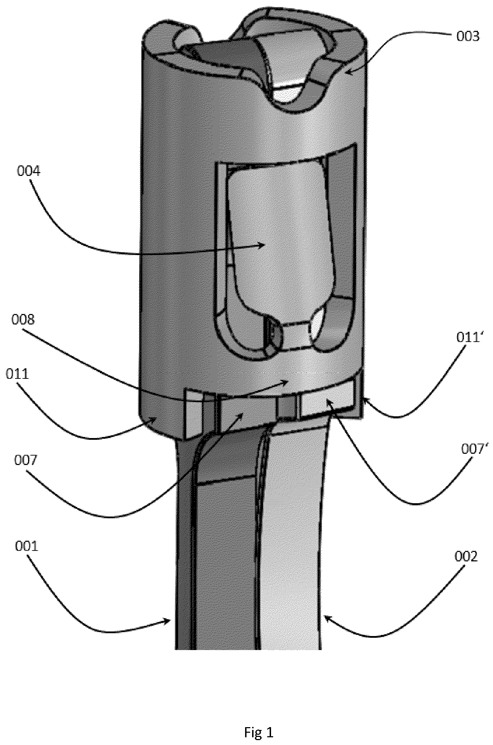

Fig. 1 illustrates one embodiment of a connecting device according to the disclosure wherein a 1^{st} strut (001) and a 2^{nd} strut (002) are placed inside the snap lock cover (003) and wherein the 1^{st} strut (001) and a 2^{nd} strut (002) are locked by snap lock tongue (004). The optional features snap lock hard stop (008) and strut hard stops (007, 007') can be advantageous to better position and lock the struts (001, 002) within the snap lock cover (003). The optional feature snap lock cover skirt (011, 011') can further provide for an even better holding of the struts (001, 002) within the snap lock cover (003). The snap lock hard stop (008) and strut hard stops (007, 007') can avoid or prevent that struts (001, 002) continue to keep on sliding within the snap lock cover (003). The snap lock cover skirt (011, 011') supports to avoiding or at least reducing twisting of the struts (001, 002) and thus prevent the unlocking of the struts.

Fig. 2 shows a longitudinal cut of a two-part heart valve replacement prosthesis in the major figure and a blow up of the struts (001, 002) from the different parts of the prosthesis not yet assembled and to be connected by the snap lock cover (003). An outer 1^{st} stent (005) and an inner 2^{nd} stent (006) are depicted wherein the blow up figure shows the respective struts (001, 002) which are aligned (see arrow) and in a next step introduced into the snap lock cover (003). The 2^{nd} stent (006) is pushed inside upwards the 1^{st} stent (005) and hence the struts (001, 002) are positioned down-to-up or up-to-down (strut 002 of 2^{nd} stent 006 is the "down strut" and strut 001 of 1^{st} stent 005 is the "up strut"). It can also be denoted in terms of the longitudinal stent/prosthesis in direction and in relationship to the operator operating the catheter device for implantation and deployment, i.e. distal is the "down direction" and thus the "down strut" and proximal is the "up direction" and thus the "up strut". Upon alignment the snap lock cover (003) is pushed over the two struts (001, 002) and locked by snap lock tongue (004) wherein the optional features snap lock hard stop (008) stop two struts (001, 002) at the correct position by way of strut hard stops (007, 007') for optimal locking of the struts (001, 002) by way of snap lock tongue (004); and the optional feature snap lock cover skirts (0011, 0011') contribute to an additional locking force on the proximal and distal direction (up and down direction). Moreover, they avoid or contribute to preventing opening of the connection and thus can contribute to a safe connection of the struts.

Fig 2a depicts the continuation of the assembling processing (locking process) of Fig. 2. The struts (001, 002) of the two stents (005, 006) are now fully aligned. The two struts (001, 002) are aligned and now the snap lock cover (003) is pushed over them / the struts (001, 002) are introduced inside the snap lock cover (003).

Fig. 2b illustrates in the blow up figure the connected stents (005, 006) wherein the struts (001, 002) are introduced inside the snap lock cover (003) and locked by snap lock tongue (004). All other optional means for a correct, safe and secure locking are in their correct positions (007, 007', 008, 011, 011').

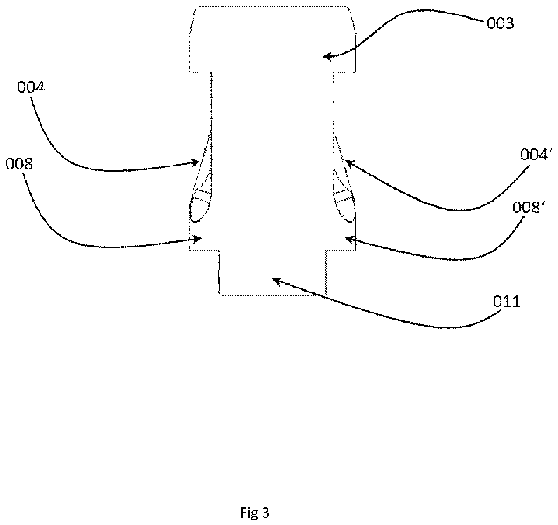

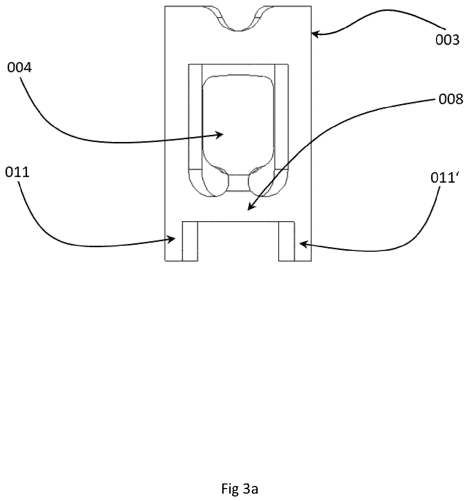

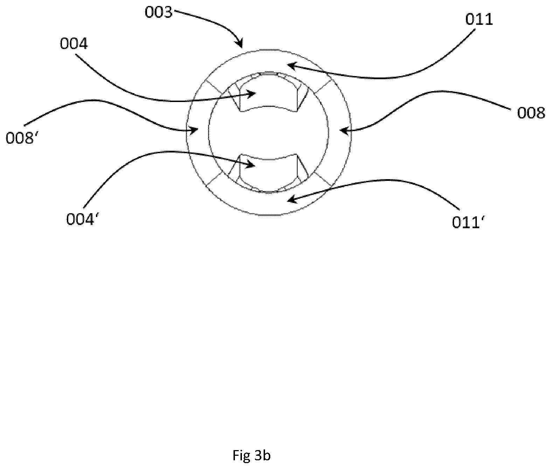

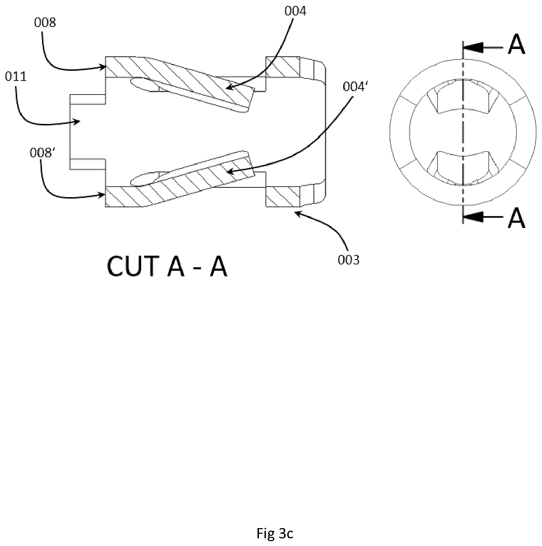

Fig. 3, 3a, 3b show different views (side and top views) of a snap lock cover according to the disclosure wherein the snap lock cover (003) comprises two snap lock tongues (004, 004') and two snap lock hard stops (008, 008').

Fig. 3c shows a longitudinal cut through a snap lock cover (003) and indicated the cut line (A-A). The snap lock tongues (004, 004') are pointing inward wherein they are flexible to such an extend that the struts (001, 002 - not shown here) can be introduced and pushed inside the snap lock cover as far as necessary and the counter parts on the struts (001, 002) will lock with the snap lock tongues (004, 004'). Depicted are also the optional features snap lock hard stop (008, 008') and snap lock cover skirt (011).

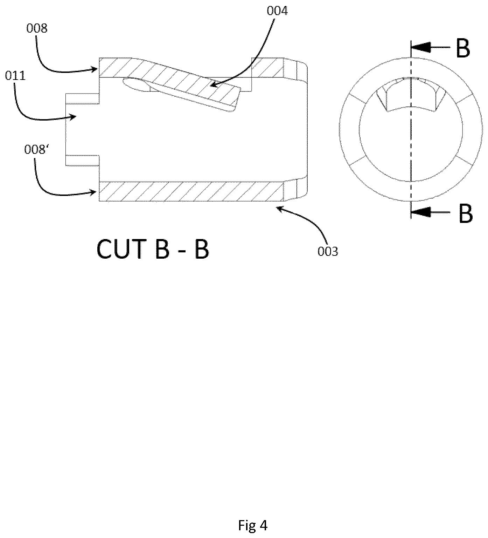

Fig. 4 depicts a variation of a snap lock cover (003) according to the disclosure wherein only one snap lock tongue (004) is used. The figure shows the top view and cut (B-B) and also the optional features (008, 008', 011).

Fig. 5 shows the detail of an alignment (see arrows) of two struts (001, 002) wherein strut tongue stops (009, 009') of the struts (001, 002) and strut hart stops (007, 007' - optional) are depicted.

Fig. 5a, 5b illustrate the assembling of the parts (001, 002, 003) for a safe and easy connection of two medical device parts. In Fig. 5a the arrows indicate the pushing of snap lock cover (003) over the struts (001, 002). In Fig. 5b the parts (001, 002, 003) are assembled and the two medical parts are safely connected to each other.

Fig. 5c shows the alignment of the two struts (001, 002) inside a snap lock cover (003) wherein the struts are aligned from left to right outside-to-inside or with the view from right to left inside-to-outside wherein the struts are tapered because of the origin of the medical parts cut from a tube, e.g. a nitinol tube, i.e. the outside of the tube is larger than the inside of the tube. In reference to the distal/proximal orientation of the device the two struts can either be superimposed in distal to proximal direction or they can be side-by-side in distal to proximal direction. The different orientations and alignments imply that the strut hard stops (007, 007'), strut tongue stops (009, 009', 009", 009‴) are correctly placed on the strut for correct alignment and introduction into the snap lock cover (003).

Fig. 5d is the side view of 5s and depicts a snap lock cover (003) with one snap lock tongue (004) a snap lock hard stop (008), two snap lock cover skirts (011, 011') and two struts (001, 002) carrying strut hard stops (007, 007'). 004' and 008' are not visible.

Fig. 6 to 8 show various embodiments of snap lock covers (003) according to the disclosure.

Fig. 6, 6a, 6b show a snap lock cover (003) in round shape form wherein two snap lock tongues (004, 004', 004", 004‴) on each side are comprised (in Fig. 6a, 6b one side is not visible) and on one side of the snap lock cover (003) a snap lock hard stop (008) and two snap lock cover skirts (011, 011') are comprised and visible. Hence the connecting means (e.g. struts of the two medical device parts) will be engaged from one side into the snap lock cover (003) for connection of the two medical device parts. Certain details are not visible as they are on the reverse side of the figure.

In particular, Fig.6 shows a snap lock cover (003) in round shape form wherein four snap lock tongues (004, 004', 004", 004‴), two snap lock hard stops (008, 008') and two snap lock cover skirts (011, 011' not visible) are comprised. Hence the connecting means (e.g. struts of the two medical device parts) will be engaged from one side into the snap lock cover (003) for connection of the two medical device parts.

Fig. 6a, shows in particular a side view of Fig.6 wherein features like two snap lock tongues (004', 004‴) and one snap lock hard stop (008') are not visible.

Fig. 6b, shows in particular an isometric view of Fig.6 wherein features like two snap lock tongues (004', 004"') and one snap lock hard stop (008') and one snap lock cover skirt (011') are not visible.

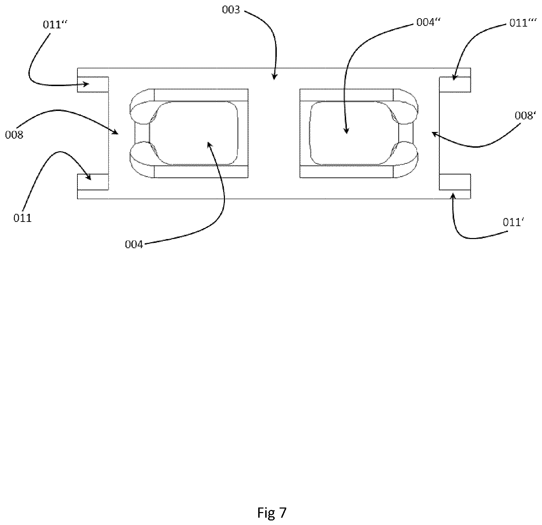

Fig. 7 to 9c show a snap lock cover (003) according to the disclosure wherein the connecting means (e.g. struts 001, 002) are introduced and engaged into the snap lock cover (003) from different sides. Each strut (001, 002) comprises two strut tongue stops (009, 009', 009", 009‴) and two strut hard stops (007, 007'). Arrows indicate the direction in which the struts (001, 002) are introduced into the snap lock cover (003).

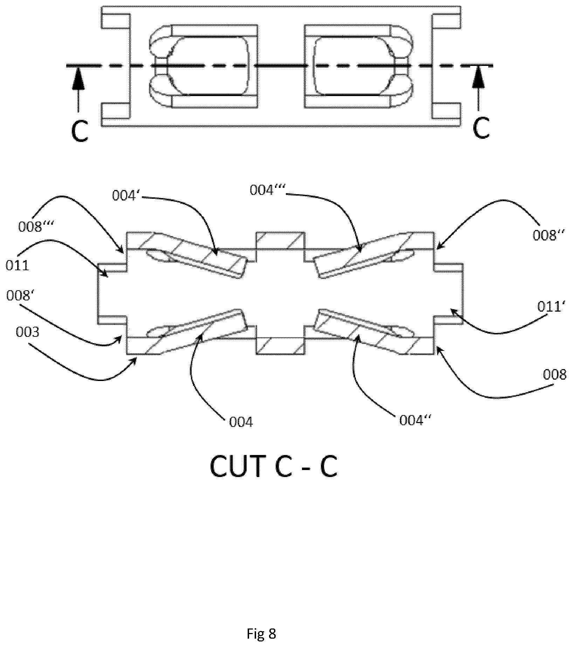

Fig. 8 shows a cut (C-C) of Fig.7 illustrating details of the snap lock tongues (004, 004', 004", 004‴) directing inwardly. Also shown are the optional features snap lock hard stop (008, 008', 008", 008"') and snap lock cover skirts (011, 011').

Fig. 9, 9a to 9c show different embodiments according to the disclosure of connecting struts of different medical device parts using a snap lock cover according to the disclosure. Thus, the strut tongue stops (009, 009', 009", 009‴) are positioned distally on the respective struts (001, 002) and lock with the snap lock tongue being further away from the side wherein the respective strut (001, 002) is introduced into the snap lock cover (003). It is also possible that only two snap lock tongues (004) are comprised in the snap lock cover (003) either on the same side or on different sides e.g. 180° opposite. Other degrees of turning the position of the snap lock tongues can be envisaged.

In Fig. 9b the fully assembled device is shown. Fig. 9c includes a cut of the connecting device (D-D) wherein the struts are shown locked with the snap lock tongues and the strut tongue stops (004, 004', 004", 004"'; 009, 009', 009", 009‴).

Fig. 10, 10a, 10b illustrates different embodiments of snap lock covers according to the disclosure wherein the snap lock cover has a rectangular shape. This may have the advantage of a very dense connection of the struts (001, 002) with the snap lock cover (003) without hollow areas. The snap lock tongue (004), snap lock cover skirts (011, 011') and snap lock hard stops (008, 008') are depicted.

Fig. 11 illustrates one embodiment of strut align means (010, 010') positioned on two struts (001, 002) which facilitate a precise alignment of two struts and correct positioning within the snap lock cover (003). The arrows indicate movement of the struts (001, 002) during assembling and connection of the struts into the snap lock cover (003 - not shown in Fig. 11). Here the orientation of the struts is that upward or downward is the outside or inside of the original tube (e.g. nitinol) which is laser cut. Hence the two struts align at the cur sides and with regard to the original tube the cut sides align wherein the outer and inner tube side face upwardly and downwardly, respectively or vise versa. With respect to the tube cut one can also denote the assembling as being side-by-side with respect to the cutting.

Fig. 11a shows the connection process of the struts (001, 002) including the strut align means (010, 010') with the snap lock cover (003) which is pushed over the struts (001, 002) and which connects the two medical device parts in an easy and safe manner. Fig. 11b depicts the fully assembled parts (001, 002, 003).

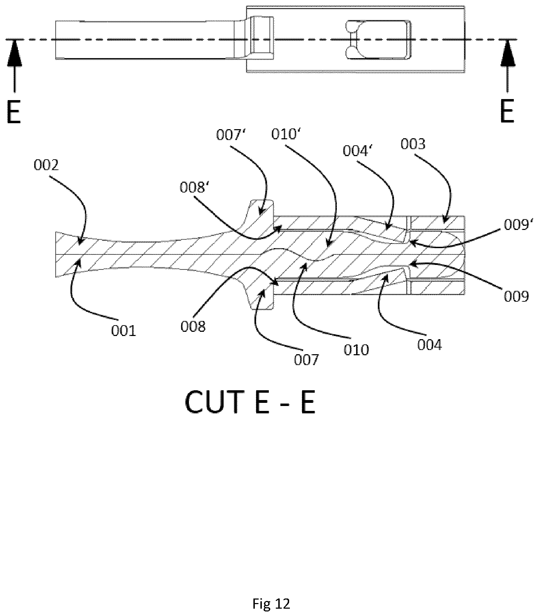

Fig. 12 is a cut (E-E) of Fig 11b showing the connected struts (001, 002) by snap lock cover (003).

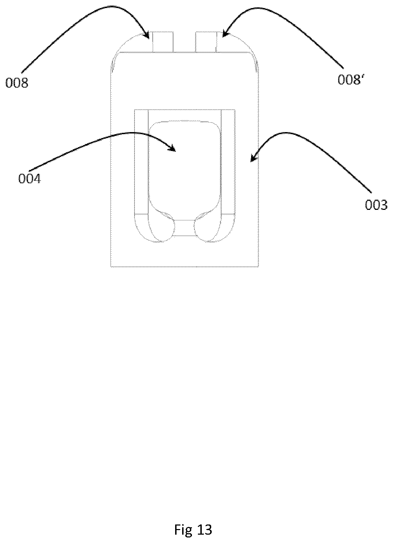

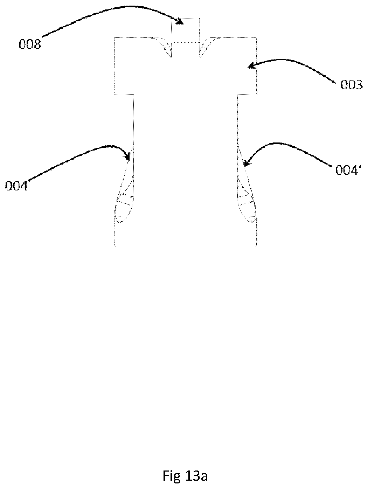

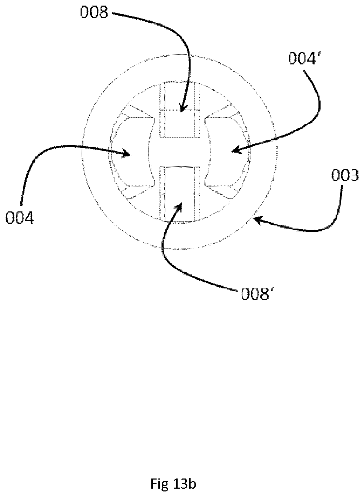

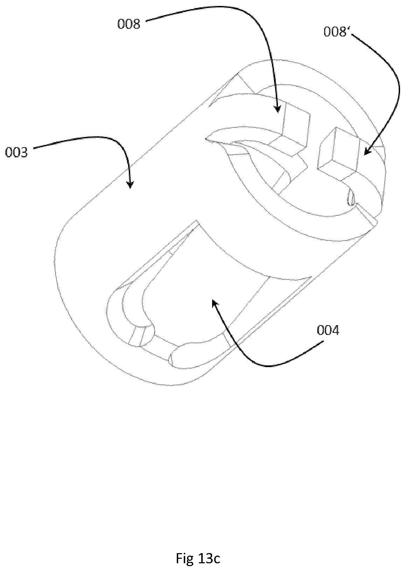

Fig. 13 illustrates a variation of a snap lock hard stop (008, 008') which is here at the distal end of the snap lock cover (003). Hence, a snap lock hard stop can be placed either at the proximal or the distal end of the snap lock cover wherein the proximal position denotes the side wherein the strut is introduced into the snap lock cover (003) and the distal position of the snap lock hard stop denotes the side opposite the introduction side of the strut into the snap lock cover (003). The snap lock hard stop can be one or several and where it is positioned opposite the strut introduction side it can be one or several snap lock hard stops (see. e.g. Fig 13, 13a, 13b, 13c).

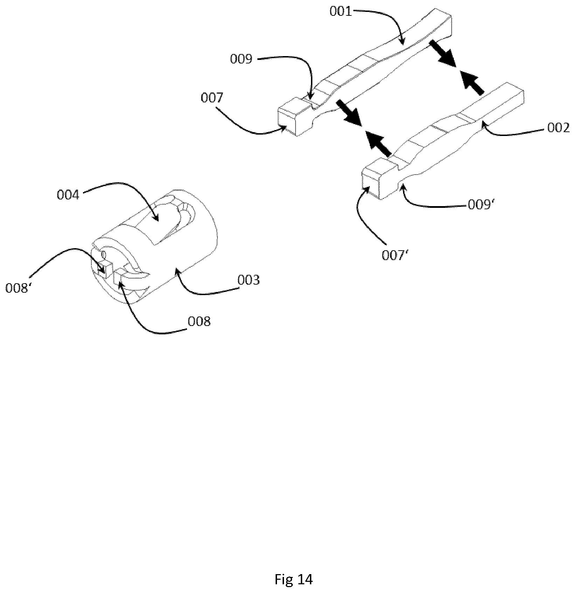

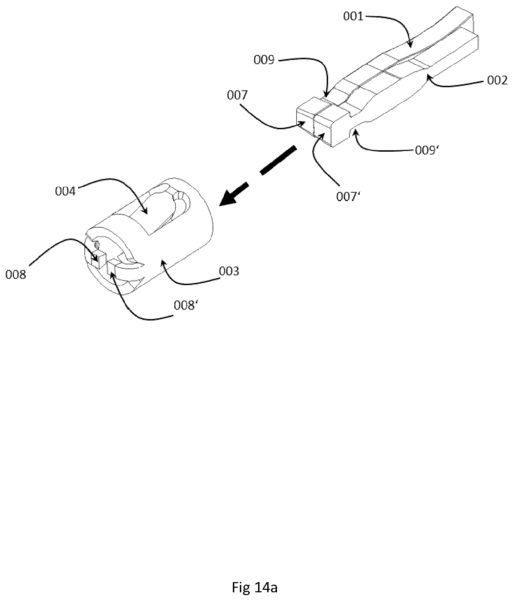

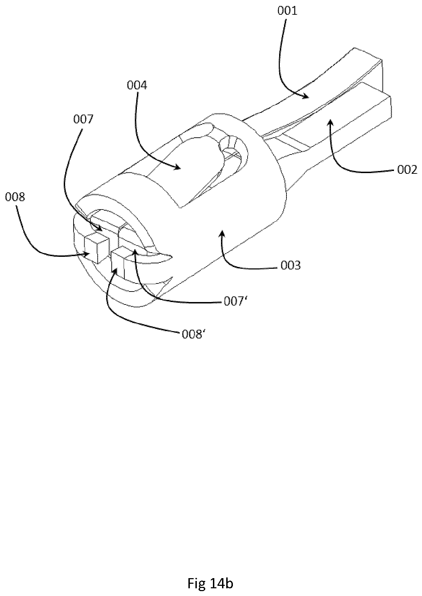

Fig. 14 depicts the snap lock cover (003) of Fig 13c together with struts (001, 002) and indicates the alignment of the struts (001, 002) by way of arrows and their assembly (Fig. 14a, 14b).

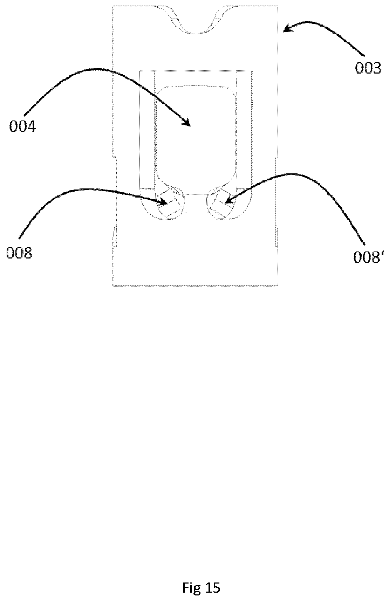

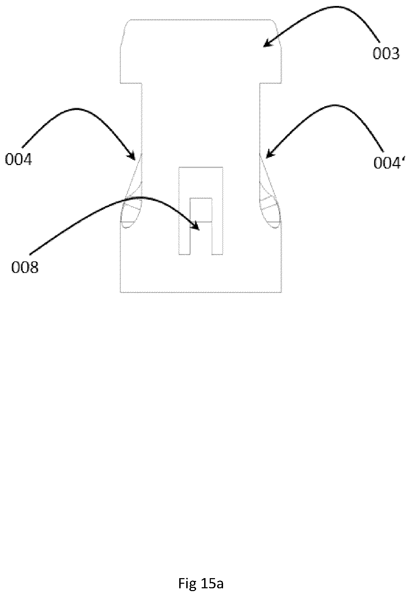

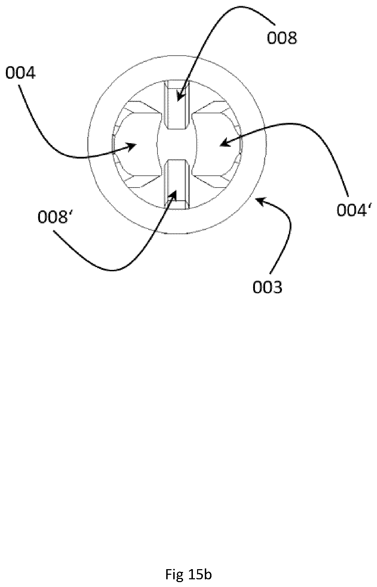

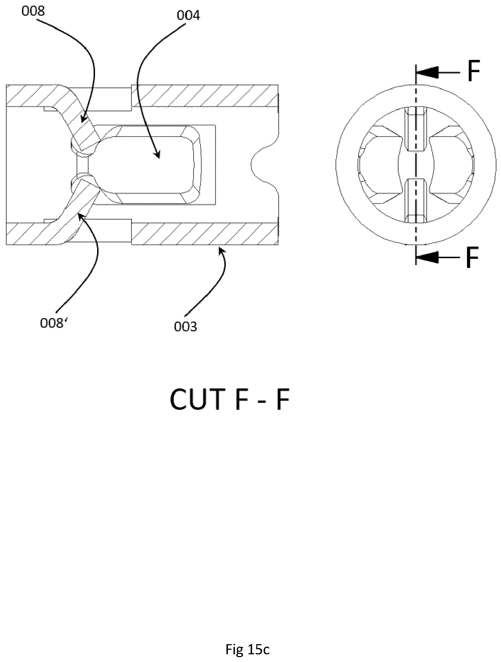

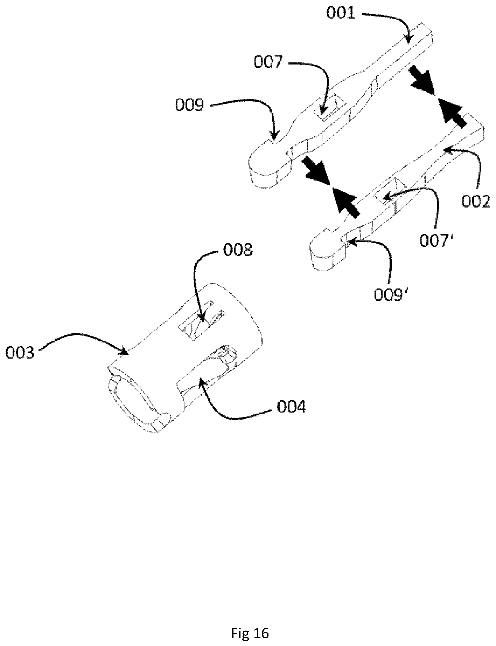

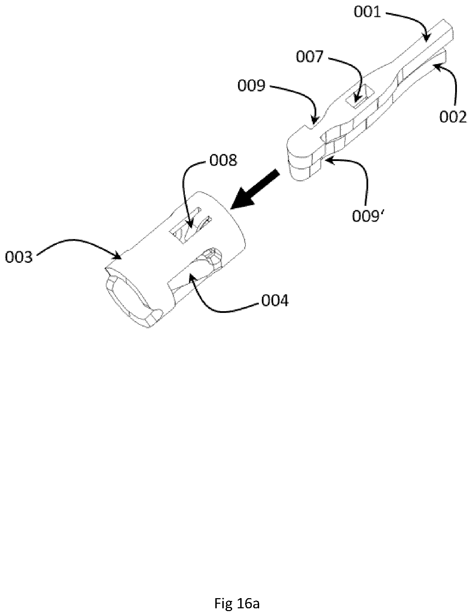

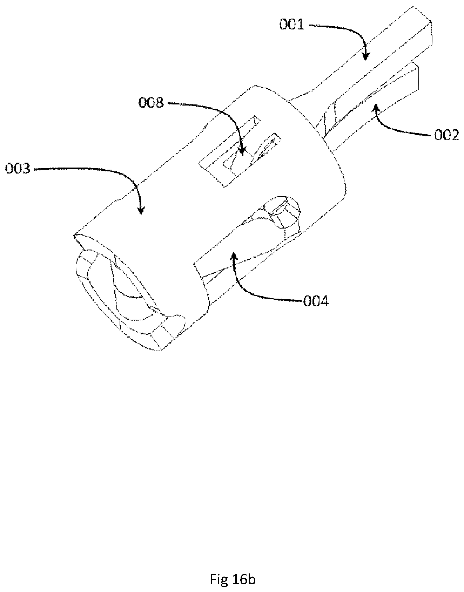

Fig. 15, 15a, 15b and 15c (cut F-F) show a variation according to the disclosure wherein the snap lock hard stop (008, 008') is positioned inside the snap lock cover (003) and finds its counterpart (strut hard stop 007, 007') in the strut (001, 002) as a special design feature within the strut as a cut or carve out as shown in Fig. 16.

Fig 16a to 16b show the assembling (see arrow) and connection of the struts (001, 002) with the snap lock cover (003) and specially designed snap lock hard stops (008).

### Reference Number List

001 - 1^{st} strut
002 - 2^{nd} strut
003 - snap lock cover
004 / 004' / 004" / 004‴ - snap lock tongue
005 - 1^{st} stent
006 - 2^{nd} stent
007 / 007' - strut hard stop
008 / 008' / 008" / 008"' - snap lock hard stop
009 / 009' / 009" / 009"' - strut tongue stop
010 / 010' - strut align means
011 / 011' - snap lock cover skirt

## Claims

1. Medical connecting device useful for connecting an inner stent and an outer stent(basis claim 6) **characterized by** engaging and locking two connecting features of said inner stent and said outer stent wherein a first connecting feature forms part of the inner stent and a second connecting feature forms part of the outer stent wherein the medical connecting device is exhibiting at least one snap lock tongue and one snap lock cover wherein the connecting features are aligned in the same or a counter direction and are locked by snap lock tongues.

2. Medical connecting device according to claim 1 wherein the snap lock cover comprises further at least one snap lock hard stop, preferably 2, 3, 4, 5, 6, 7, or 8, and/or at least one snap lock cover shirt, preferably 2, 3, or 4.

3. Medical connecting device according to claim 1 or 2 wherein one or several snap lock hard stops are positioned at the proximal end, or at the distal end or/and in a region between the distal and proximal end of the snap lock cover.

4. Medical connecting device according to claim 1, 2 or 3 wherein the snap lock cover is round or square-cut or angular in shape or/and longitudinal in shape.

5. Two part stent comprising an inner stent and an outer stent wherein each stent comprises at least one connecting features wherein one connecting feature of the first stent and one connecting feature of the second stent are aligned in the same direction or in a counter direction and a snap lock cover is engaging the two connecting features and locking the two connecting features by way of at least one snap lock tongue, preferably one or two snap lock tongues, and at least one strut tongue stop of each connecting feature.

6. Heart valve replacement prosthesis comprising a replacement valve and a two part stent comprising an inner stent and an outer stent wherein each stent comprises at least one connecting feature wherein one connecting feature of the first stent and one connecting feature of the second stent are aligned in the same direction or in a counter direction and a snap lock cover is engaging the two connecting features and locking the two connecting features by way of at least one snap lock tongue and one strut tongue stop of each connecting feature.

7. Two part stent or heart valve replacement prosthesis according to claims 5, or 6 wherein the connecting feature(s) of the stent is (are) directing inwardly in an angle with regard to the longitudinal direction of the inner part or stent of about 3° to 60°, preferably of 5° - 30°, more preferably 25°.

8. Two part part stent or heart valve replacement prosthesis according to claims 5, 6, or 7 wherein the connecting feature(s) of the outer part or stent is (are) superimposed or side-by-side in relation to the proximal to distal orientation or aligned inside-to-outside, or inside-to-inside, or outside-to-outside with reference to the original tube from which the part or stent was cut, or aligned tube cut edge to tube cut edge with reference to the original tube from which the part or stent was cut.

9. Two part part stent or heart valve replacement prosthesis according to any of claims 5 to 8 comprising one snap lock cover and 2, 3 or 4 snap lock tongues and the respective numbers of strut tongue stops of the connecting features.

10. Two part stent or heart valve replacement prosthesis according to any of claims 5 to 10 comprising 1 snap lock hard stop and one strut hard stop of each connecting feature or, 2, 3 or 4 snap lock hard stops and 1 or 2 strut hard stops of each connecting feature.

11. Two part stent or heart valve replacement prosthesis according to any of claims 5 to 10 wherein the snap lock tongue(s) is/are positioned in longitudinal direction of the medical device, stent or prosthesis or essentially 90° to the longitudinal direction or in an angle of between 5° and 90°, or 10° to 45° or 45° to the longitudinal direction.

12. Two part part stent or heart valve replacement prosthesis according to any of claims 5 to 11 wherein the connecting feature is positioned in the distal area or at the distal end of a strut extending from each part of the medical device, stent or prosthesis.

13. Two part part stent or heart valve replacement prosthesis according to any of claims 5 to 12 wherein the connecting feature or each connecting feature comprises at least one strut align means, preferably wherein two strut align means of connecting feature pairs.

14. Two part stent or heart valve replacement prosthesis comprising at least one medical connecting device according to any of claims 1 to 4.
